# EUROPEAN PATENT SPECIFICATION

(11) **EP 2 695 572 B1**
(45) Date of publication and mention of the grant of the patent: **20.01.2016**
(21) Application number: 13179237.6
(22) Date of filing: 05.08.2013
(51) Int. Cl.: A61B 3/135

(54) **Slit lamp microscope**
Spaltlampenmikroskop
Microscope de lampe à fente

(30) Priority: 07.08.2012 JP 2012175277
(43) Date of publication of application: 12.02.2014
(73) Proprietor: Kabushiki Kaisha Topcon, Tokyo 174-8580 (JP)
(72) Inventor: Watanabe, Takahiro, Tokyo 174-8580 (JP); Takeda, Takanori, Tokyo 174-8580 (JP)
(74) Representative: Vossius & Partner Patentanwälte Rechtsanwälte mbB

(56) References cited:
- EP-A1- 2 248 460
- WO-A1-2012/172907
- JP-A- 2007 047 657
- US-A1- 2009 314 749

## Description

### [Field of the Invention]

The present invention relates to a slit lamp microscope.

### [Background of the Invention]

A slit lamp microscope is an ophthalmologic apparatus that uses slit light to cut an optical section of a cornea, thereby obtaining an image of a cross-section of the cornea. The slit lamp microscope is provided with a pair of slit blades for obtaining the slit light. The user appropriately adjusts the interval between the slit blades (slit width) and conducts observation.

### [Prior Art Documents]

### [Patent Documents]

[Patent Document 1] Japanese Published Unexamined Application 2009-178459

EP 2 248 460 A describes that the examiner can regulate the slit width as necessary or a controller can change the slit width to a predetermined default value.

JP 2007-47657 A relates to a mechanism having stepping motors each for driving slit members, thereby adjusting a slit width between the slit members. A controller is adapted to receive a variable command value. The controller applies a predetermined pulse number to the stepping motors on the basis of the received command value. The controller comprises a correction means for correcting the pulse number to ensure that the slit width has a proportional relation with the command value. The correction means is provided with a storage for storing corrected pulse numbers in association with respective command values.

US 2009/0314749 A discloses producing a slit width table in which a displacement amount of the slit width from an original point and a command value correspond to each other. The slit width table is produced in advance in the manufacturing process of a microscope laser processing apparatus.

WO 2012/172907, which may form the state of the art under Art. 54(3) EPC, discloses a slit lamp microscope which allows to appropriately and easily carry out setting of the optical system of the microscope. Reference setting conditions are stored in advance with respect to each of a plurality of sites of an eye. When a site of the eye is designated, a setting state identification unit of the microscope identifies items such as magnification, slit width, light intensity of the microscope, the current setting states of which are different from the reference setting conditions that are associated with the designated site of the eye.

### [Problem to be Solved by the Invention]

During an examination using a slit lamp microscope, there are cases in which it is desired to carry out observations by repeatedly applying the same slit width. For example, in the case of observing an eye while changing the slit width (and illumination direction) during a single examination, there are times in which it is desired to carry out observations using the same slit width repeatedly. Moreover, for follow-up and preoperative/postoperative observations, there are cases in which it is desired to carry out observations by reproducing the slit width used in past examinations.

In such cases, the slit width was adjusted each time with the conventional slit lamp microscope. However, slit width adjustment must be carried out with a high degree of accuracy, and doing so each time is troublesome, in addition to prolonging examinations.

The object of the present invention is to provide a slit lamp microscope capable of simple observation using the same slit width.

### [Means for Solving the Problem]

The aforementioned object is achieved with the features of the claims.

### [Effect of the Invention]

According to the slit lamp microscope related to the present invention, it is possible to easily carry out observations using the same slit width.

### [Brief Description of the Drawings]

Fig. 1 is a schematic side view illustrating an example of the exterior configuration of the slit lamp microscope related to the embodiment.
Fig. 2 is a schematic side view illustrating an example of a configuration of the optical system of the slit lamp microscope related to the embodiment.
Fig. 3 is a schematic block diagram illustrating an example of a configuration of the control system of the slit lamp microscope related to the embodiment.
Fig. 4 is a flow chart illustrating an example of an operation of the slit lamp microscope related to the embodiment.
Fig. 5 is a flow chart illustrating an example of an operation of the slit lamp microscope related to the embodiment.

### [Mode for Carrying Out the Invention]

An example of the embodiment of the slit lamp microscope related to the present invention is explained in detail with reference to the diagrams. It should be noted that contents of the document presented in this specification may be appropriately incorporated as contents of the embodiment below.

First, the directions are defined. The direction from the optical element positioned closest to the subject side in a device optical system is a "front direction", while the direction opposite from this is "rear direction". Moreover, the horizontal direction orthogonal to the front direction is a "left-right direction". Further, the direction orthogonal to both the front-rear direction and the left-right direction is a "vertical direction (upward-downward direction)".

### [Exterior configuration]

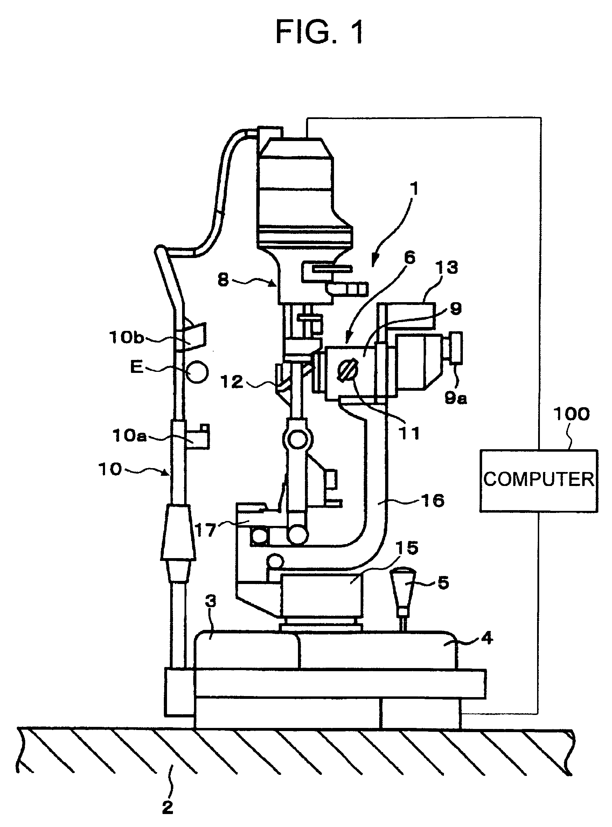

The exterior configuration of the slit lamp microscope related to the present embodiment is explained with reference to Fig. 1. A computer 100 is connected to the slit lamp microscope 1. The computer 100 carries out various control processes and arithmetic processes. Further, rather than providing a computer 100 separate from a main body of the microscope (a case for housing an optical system, etc.), a configuration is possible in which a similar computer is arranged within the main body of the microscope.

The slit lamp microscope 1 is placed on a table 2. It should be noted that the computer 100 may be arranged on the other table or other locations. A base 4 is configured to be movable in the horizontal direction via a moving mechanism 3. The base 4 moves by the tilting operation of an operation handle 5.

The top surface of the base 4 is provided with a supporting part 15 that supports an observation system 6 and an illumination system 8. A supporting arm 16 for supporting the observation system 6 is provided on the supporting part 15 such that it is capable of rotating in the left-right direction. A supporting arm 17 supporting the illumination system 8 is provided on the top part of the supporting arm 16 such that it is capable of rotating in the left-right direction. The supporting arms 16 and 17 are independently turnable around the same axis.

The observation system 6 moves by manually turning the supporting arm 16. The illumination system 8 moves by manually turning the supporting arm 17. It should be noted that the respective supporting arms 16 and 17 may be configured so as to be turned by an electric mechanism. In such cases, an actuator generating a driving force for turning the respective supporting arms 16 and 17 and a transmission mechanism transmitting this driving force are provided. The actuator is configured by, for example, a pulse motor. The transmission mechanism is configured by, for example, a combination of gearwheels, or rack and pinion, etc.

The illumination system 8 projects illumination light onto an eye E. The illumination system 8 may be, as mentioned above, swung in the left-right direction around a vertically extending turning axis. Thereby, the projection direction of the illumination light with respect to the eye E changes. The illumination system 8 may be configured such that it is allowed to be swung vertically. That is, it may be configured such that the angle of elevation and the angle of depression of the illumination light can be changed.

The observation system 6 comprises a left-and-right pair of optical systems for guiding a returned light of the illumination light from the eye E. These optical systems are housed within a lens tube body 9. The end of the lens tube body 9 is an eyepiece 9a. The user observes the eye E by looking into the eyepiece 9a with the naked eyes. The lens tube body 9 may be turned in the left-right direction by means of turning the supporting arm 16 as mentioned above. Thereby, the direction of the observation system 6 with respect to the eye E may be changed. It should be noted that the returned light of the illumination light includes a variety of light components passing the eye E such as scattered light, and with this variety of light components collectively referred to as "returned light."

A jaw holding platform 10 is arranged in a position facing the lens tube body 9. The jaw holding platform 10 is provided with a jaw holder 10a and a forehead supporter 10b for stably placing the subject face.

An observation magnification operating knob 11 for changing the observation magnification is arranged on the side surface of the lens tube body 9. Further, an imaging device 13 for imaging the eye E is connected to the lens tube body 9. The imaging device 13 comprises an image sensor. The image sensor is a photoelectric transducer that detects light and outputs electric signals (image signals). The image signals are input into the computer 100. For the image sensor, for example, a CCD (Charge Coupled Device) image sensor or a CMOS (Complementary Metal Oxide Semiconductor) image sensor is used. A mirror 12 for reflecting illumination light beam output from the illumination system 8 towards the eye E is arranged in a lower position of the illumination system 8.

### [Configuration of the optical system]

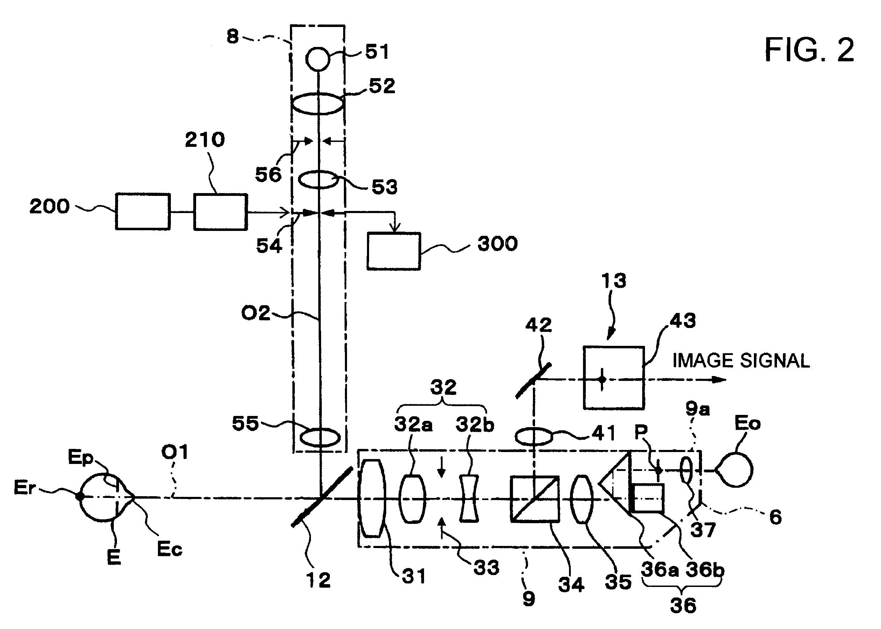

The configuration of the optical system of the slit lamp microscope 1 is described with reference to Fig. 2. The slit lamp microscope 1 comprises the observation system 6 and the illumination system 8.

### [Observation system]

The observation system 6 comprises a left-and-right pair of optical systems. The left and right optical systems have substantially the same configuration. By means of these left and right optical systems, the user is capable of observing the eye E with both eyes. It should be noted that Fig. 2 illustrates only one of the left and right optical systems of the observation system 6. The symbol O1 is the optical axis (observation optical axis) of the observation system 6.

Each of the left and right optical systems of the observation system 6 comprises an objective lens 31, magnification optical system 32, diaphragm 33, relay lens 35, prism 36, and eyepiece lens 37. The beam splitter 34 is provided to only one or both of the left and right optical systems. The eyepiece lens 37 is provided inside the eyepiece 9a. The symbol P indicates the imaging position of the light guided towards the eyepiece lens 37. The symbol Ec indicates the cornea of the eye E, the symbol Ep indicates the iris, and the symbol Er indicates the fundus, respectively. The symbol Eo indicates the user's eye.

The magnification optical system 32 comprises a plurality (for example, two) of magnifying lens 32a and 32b. Each magnifying lens 32a and 32b is movable along the observation optical axis O1. Thereby, the magnification (angle of view) of observation images and photographed images of the eye E may be changed. Changing the magnification is carried out by operating the observation magnification operating knob 11. Moreover, a configuration is possible wherein the magnification is electrically changed using a switch (not illustrated), etc.

The beam splitter 34 divides the light travelling along the observation optical axis O1 into two. The light that has penetrated the beam splitter 34 is guided to the user's eye Eo via the prism 36, and the eyepiece lens 37. The prism 36 comprises two optical elements 36a and 36b, and parallelly displaces the travelling direction of the light upwards.

Meanwhile, the light that has been reflected by the beam splitter 34 is guided to the image sensor 43 of the imaging device 13 via the relay lens 41 and the mirror 42. The image sensor 43 detects this reflected light to generate image signals.

### [Illumination system]

The illumination system 8 comprises a light source 51, relay lens 52, illumination diaphragm 56, condensing lens 53, slit forming part 54, and condensing lens 55. The symbol 02 indicates the optical axis (illumination optical axis) of the illumination system 8.

The light source 51 outputs illumination light. It should be noted that a plurality of light sources may be provided in the illumination system 8. For example, both a light source outputting a continuous light (halogen lamp, LED, etc.) and a light source outputting a flash light (xenon lamp, LED, etc.) may be provided as the light source 51. Moreover, a light source for cornea observation and a light source for eye-fundus observation may be separately provided.

The slit forming part 54 is used for generating slit light. The slit forming part 54 comprises a pair of slit blades. By changing the interval of these slit blades, the slit width changes.

The slit blades shift by means of an actuator 200 and a driving mechanism 210. The actuator 200 is a device that generates a driving force, and is configured by, for example, a pulse motor. The driving mechanism 210 changes the interval between the slit blades based on the driving force generated by the actuator 200, which corresponds to a "mechanism."

A detector 300 is provided in the vicinity of the slit forming part 54. The detector 300 detects the event in which the slit blades are positioned in the standard position. The detector 300 is, for example, a photo sensor. The photo sensor is a device comprising a light-emitting element and a light-receiving element, detecting the change of intensity of light depending on a detection object (the slit blades) and outputting electric signals.

The illumination diaphragm 56 is configured such that the size of the light-transmitting part thereof may be changed. The illumination diaphragm 56 is particularly effective for eye-fundus observation. For example, the illumination diaphragm 56 is used for reducing reflection of the illumination light by the cornea Ec and/or crystalline lens and adjusting the brightness of the illumination light.

### [Configuration of control system]

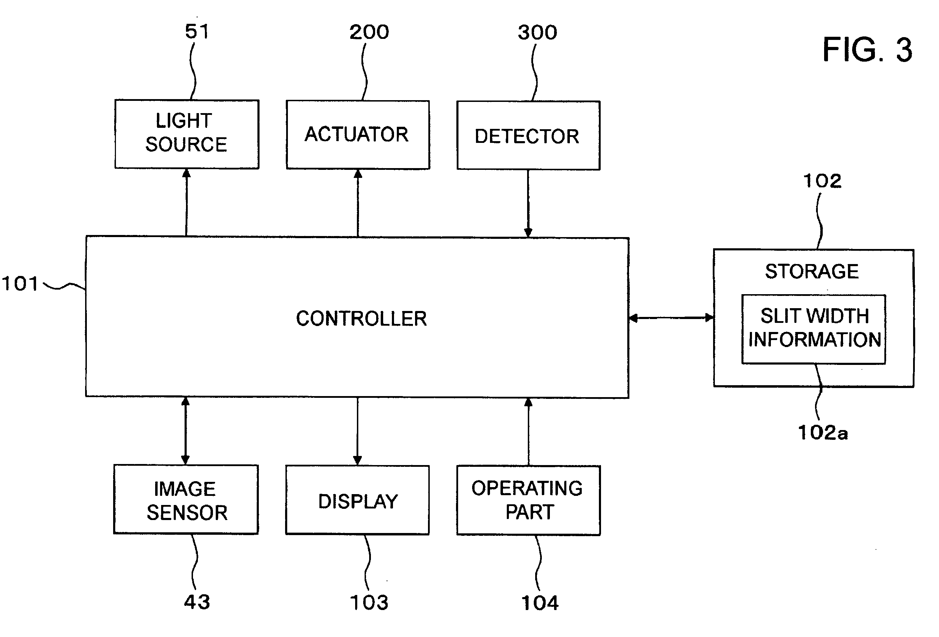

The control system of the slit lamp microscope 1 is explained with reference to Fig. 3. The control system of the slit lamp microscope 1 is configured with a controller 101 as the center thereof. Further, Fig. 3 illustrates only the particularly focal components in this embodiment, with other components omitted.

### [Controller]

The controller 101 controls each part of the slit lamp microscope 1. For example, the controller 101 carries out control of the observation system 6, control of the illumination system 8, etc. the control of the observation system 6 includes control of the magnification optical system 32, control of the diaphragm 33, and control of the charge storage time, sensitivity, frame rate, etc. of the image sensor 43. The control of the illumination system 8 includes control of the light source 51, control of the illumination diaphragm 56, and control of the slit forming part 54. It should be noted that the control of the slit forming part 54 is carried out by controlling the actuator 200. If the actuator 200 is a pulse motor, the controller 101 transmits pulse signals to the actuator 200. Moreover, the controller 101 is input with electrical signals from the detector 300. Thereby, the controller 101 detects that the slit blades are in the standard position. Moreover, the controller 101 carries out a process of reading data stored in the storage 102 and a process of writing data into the storage 102. Moreover, a device other than a pulse motor, such as a direct current motor, may be used as the actuator 200. In this case, a potentiometer or an encoder may be used as the sensor (the detector 300) that detects the position of the slit blades or the state of operation of the actuator.

The controller 101 comprises hardware such as a microprocessor, RAM, ROM, hard disk drive, etc. A control program is stored in advance in this hard disk drive. Operations of the controller 101 are realized by cooperation of the control program and the aforementioned hardware.

The controller 101 is arranged in the main body (for example, in base 4) of the slit lamp microscope 1 and/or the computer 100.

### [Storage]

A variety of information is stored in the storage 102. Specifically, slit width information 102a is stored in the storage 102. The slit width information 102a includes one or more pieces of information each indicating an interval (slit width) between the pair of slit blades configuring the slit forming part 54. Information indicating the slit width is an example of "interval information." A method for generation and a method for usage of the interval information are described later. Further, the interval information may be information generated according to the method mentioned later or may be information indicating default values of the slit width. As the default value of the slit width, for example, a value obtained by statistically processing the clinically obtained slit width data (mean value, median value, mode, etc.) may be used.

The storage 102 may store distance information in advance. The distance information indicates the distance between the slit blade position when the slit blades of the slit forming part 54 are closed and the standard position mentioned above. This distance does not need to be a special distance and should he a quantity equivalent to the distance. For example, if the actuator 200 is a pulse motor, the distance information may be the number of pulses of the pulse signal transmitted from the controller 101 to the pulse motor.

The distance information may be generated, for example, by the following processes: causing the detector 300 to detect the event in which the slit blades are located in the standard position; inputting pulse signals into the pulse motor from this state until the slit blade closes; counting the number of pulses of the input pulse signals. The distance information is made, for example, prior to product shipment of the slit lamp microscope 1. Moreover, the distance information may be revised after commencing use of the slit lamp microscope 1.

### [Display]

The display 103 is controlled by the controller 101 to display a variety of information. The display 103 comprises any kinds of display device, for example, a flat panel display such as LCD, etc., or a CRT display. The display 103 may be provided on the main body of the slit lamp microscope 1 or on the computer 100.

### [Operating part]

The operating part 104 comprises an operation device and/or an input device. The operating part 104 comprises buttons and switches provided on the main body (for example, an operation handle 5, etc.) and a mouse, keyboard, etc. of the computer 100. Moreover, it is possible to use any operation devices and input devices such as a trackball, dedicated operation panel, switch, button, dial, etc.

In particular, the operating part 104 comprises one or more specified buttons (slit width specifying buttons). Each slit width specifying button is associated with one piece of interval information included in the slit width information 102a. For example, if three slit width specifying buttons are provided, the i-th slit width is associated with the i-th slit width specifying button (i=1, 2, 3). This association is carried out by the controller 101. As a specific example, the controller 101 manages this association using table information that associates each slit width specifying button with interval information. It should be noted that, as mentioned later, this association may be arbitrarily changed.

Such slit width information 102a may be created and stored for each patient and/or each eye. In this case, the slit width information 102a is associated with patient identification information (patient ID) and/or eye identification information (information indicating the left eye/right eye) and stored in the storage 102. For example, if the patient ID is input and/or the eye is specified (that is, specification of the left eye/right eye is carried out) using the operating part 104, the controller 101 searches, from the storage 102, the slit width information 102a associated with this patient and/or this eye based on the specified information. Then, the controller 101 carries out the processes (as stated below) using the searched slit width information 102a.

Moreover, a pushing operation and long-pushing operation may be carried out with respect to each slit width specifying button. The long-pushing operation is an operation carried out by pushing the slit width specifying button for a longer period of time than the pushing operation (for example, several seconds; this is set in advance), and is the operation for assigning the slit width information 102a with respect to the button pushed for a long period of time.

The slit width specifying button may be a hardware key or a software key. If it is a hardware key, a general button may be used as the slit width specifying button. If it is a software key, a configuration is possible wherein, for example, a GUI (graphical user interface) comprising the slit width specifying buttons is displayed on the display 103 and the operating part 104 (pointing device) is used to operate the slit width specifying buttons by clicking. If the duration time of this clicking operation is shorter than the specified time, this operation corresponds to the pushing operation mentioned above, while if this is longer than the specified time, this operation corresponds to the long-pushing operation mentioned above. It should be noted that a concrete configuration of the operating part 104 is arbitrary, such as separately providing buttons accepting the pushing operation and buttons accepting long-pushing operation, etc.

In Fig. 3, the display 103 and the operating part 104 are separately illustrated; however, these may be integrally configured. As a detailed example thereof, a touch panel LCD may be used.

### [Operation]

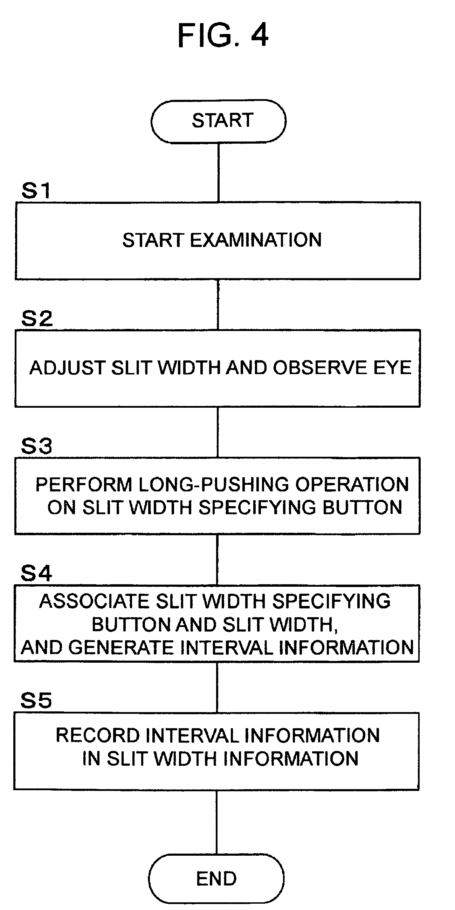

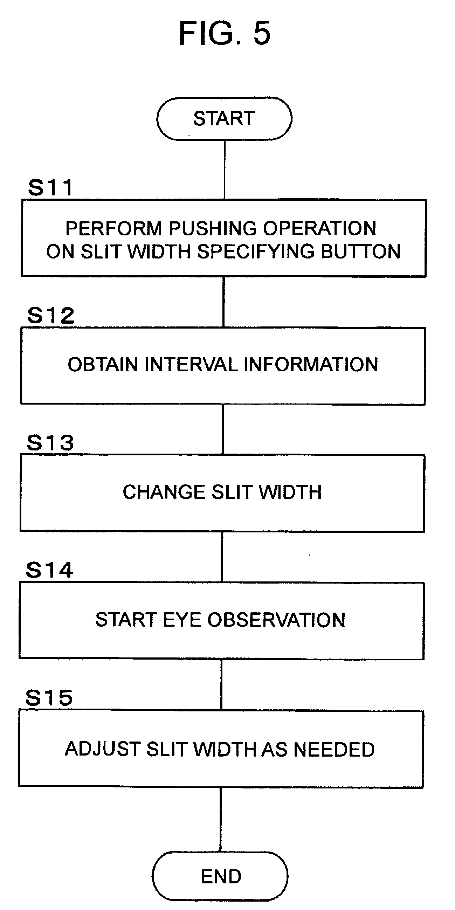

The operation of the slit lamp microscope 1 is explained. An example of the operation of the slit lamp microscope 1 is illustrated in Fig. 4 and Fig. 5. The operation example illustrated in Fig. 4 is an example of a preset process for associating slit width (interval information) to the slit width specifying buttons. The operation example illustrated in Fig. 5 is an example of a slit width reproduction process for reproducing the preset slit width.

### [Preset processing of the slit width]

### (S1: Start examination)

The user commences the examination using the slit lamp microscope 1.

### (S2: Adjust slit width and observe eye)

The user operates the operating part 104 to adjust the slit width and observes the cornea Ec of the eye E.

### (S3: Perform long-pushing operation on slit width specifying button)

In this state, the user performs the long-pushing operation with respect to any of the slit width specifying buttons.

### (S4: Associate slit width specifying button and slit width, and generate interval information)

The controller 101 associates the slit width at the time with respect to the slit width specifying button that has underwent the long-pushing operation, thereby generating the interval information.

This process includes a process that obtains the slit width at the time of performing the long-pushing operation. This slit width obtaining process may be carried out by, for example, the controller 101 obtaining the position of the slit blades based on the electric signals from the detector 300. Moreover, it is possible to obtain the slit width at the current time based on the control history of the actuator 200 (such as a transmission history of the number of pulses of pulse signals) from the time at which the standard position is detected using the detector 300 (or, in accordance with this, the time at which the slit blades are transferred into the closed state, based on the distance information) until the current time. The controller 101 associates the slit width obtained in this manner with the slit width specifying button that has underwent the long-pushing operation at Step S3, thereby generating the interval information.

### (S5: Record interval information in slit width information)

The controller 101 records the generated interval information within the slit width information 102a. It should be noted that if a slit width is already associated with respect to this slit width specifying button, it is updated to the interval information newly generated at this time. That is, the previous interval information is overwritten by the new interval information. This concludes the preset processing of the slit width.

### [Slit width reproduction process]

### (S11: Perform pushing operation on slit width specifying button)

Upon commencement of the examination or during the examination, the user carries out the pushing operation with respect to a desired slit width specifying button.

In order to simplify this pushing operation, a configuration may be adopted for presenting what slit width is associated with which slit width specifying button. As this configuration, if the slit width specifying buttons are hardware keys, it is possible to adapt a configuration of, for example, providing a device (LCD, etc.) for presenting the slit width information (slit width value, examination items, time and date of examination, etc.) on the surface of the hardware keys or in the vicinity thereof, or a configuration of displaying the information indicating the association on the display 103. Moreover, if the slit width specifying buttons are software keys, it is possible to adapt a configuration of, for example, displaying the information indicating the slit width on the slit width specifying buttons or the vicinity thereof.

### (S12: Obtain interval information)

The controller 101 obtains the interval information associated with the slit width specifying button specified at Step S11 from among the slit width information 102a.

### (S 13: Change slit width)

The controller 101 controls the actuator 200 based on the interval information obtained at Step S12, thereby changing the interval of the pair of slit blades of the slit forming part 54 to the slit width indicated in this interval information.

At this time, the detection outcome from the detector 300 and the number of pulses may be referred to in the same manner as in the preset processing. For example, a configuration is possible in which the controller 101 specifies the slit blade position (slit width) at the current time based on electric signals from the detector 300, obtains the difference between the specified slit width and the slit width indicated in the interval information, generates control signals (pulse signals, etc.) based on this difference, and transmits this to the actuator 200. Moreover, a configuration is possible in which the controller 101 moves the slit blades until the detector 300 detects that the slit blades are in the standard position, controls the actuator 200 based on the distance information to close the slit blades, generates pulse signals by determining the number of pulses for switching from this closed state to a state of the slit width indicating the interval information, and transmits the pulse signals to the actuator 200.

### (S14: Start eye observation)

The user observes the cornea Ec of the eye E with the slit width realized at Step S13.

### (S15: Adjust slit width as needed)

The user is capable of adjusting the slit width at will by means of operating the operating part 104. Specifically, if the user carries out the operation for slit width adjustment using the operating part 104, the electric signals indicating this operation content (operation signals) are input from the operating part 104 to the controller 101. The controller 101 generates control signals corresponding to the operation signals (such as pulse signals with the number of pulses corresponding to the operation signals) and transmits these control signals to the actuator 200. Thereby, the slit width changes by the amount corresponding to the operation content. This concludes the slit width reproduction process.

### [Effect]

The effect of the slit lamp microscope 1 is explained.

The slit lamp microscope 1 comprises the light source 51, the pair of slit blades (slit forming part 54), the actuator 200, the driving mechanism 210, the storage 102, the operating part 104, and the controller 101. The light source 51 emits illumination light. The slit blades generate slit light from the illumination light. The actuator 200 generates a driving force. The driving mechanism changes the interval of the slit blades (slit width) based on this driving force. The storage 102 stores one or more pieces of interval information each indicating slit width. The controller 101 controls the actuator 200 such that, when the user specifies one from among the one or more pieces of interval information using the operating part 104, the slit width becomes the interval indicated in the specified interval information.

According to such a slit lamp microscope 1, it is possible to store one or more slit widths in advance and, in accordance with the user specifying the desired slit width, automatically reproduce this slit width. Accordingly, observation using the same slit width may be easily carried out.

When the user carries out a predetermined operation using the operating part 104, the controller 101 is able to generate the interval information indicating the slit width at the time and store this in the storage 102.

Thereby, a slit width applied at any point of time may be easily stored as interval information.

The operating part 104 may comprise one or more slit width specifying buttons. In this case, the operation for specifying the interval information may be a pushing operation of a desired slit width specifying button. Further, a predetermined operation for storing the slit width at the moment may be a long-pushing operation.

According to this configuration, the operation for specifying the slit width (first operation; for example, a button pushing operation) and the operation for storing the slit width (second operation differing from the first operation; for example, long-pushing operation of the button) may be easily carried out. Moreover, both operations may be carried out with the same slit width specifying button, making simplification of the device configuration also possible.

Moreover, as the data organization of the slit width information 102a, it is possible to apply a configuration wherein the interval information and patient identification information are associated with each other. Thereby, the interval information stored for each patient may be selectively recalled and used. Moreover, the data organization may be applied in which both patient identification information and eye identification information (information indicating left eye/right eye) are associated with interval information. Thereby, interval information stored regarding each eye of each patient may be selectively recalled and used.

In the embodiment above, the photo sensor is used as the detector 300; however, any other detection device may be used. For example, a position sensor using infrared light, an encoder detecting the position of the slit blades (or parts connected to them), etc. may be used as the detector 300.

The configuration explained above is only a specific example for implementing the claimed invention.

### [Explanation of Symbols]

- 1: slit lamp microscope
- 6: observation system
- 8: illumination system
- 51: light source
- 54: slit forming part
- 100: computer
- 101: controller
- 102: storage
- 102a: slit width information
- 103: display
- 104: operating part
- 200: actuator
- 210: driving mechanism
- 300: detector
- E: eye

## Claims

1. A slit lamp microscope (1), comprising:
a light source (51) configured to emit illumination light;
a pair of slit blades configured to generate slit light from the illumination light;
an actuator (200) configured to generate a driving force;
a mechanism (210) configured to change the interval between the slit blades based on the driving force;
a storage (102) configured to store one or more pieces of interval information each indicating an interval between the slit blades;
an operating part (104); and
a controller (101) configured to, when a user specifies one from among the one or more pieces of interval information using the operating part (104), control the actuator (200) such that the interval between the slit blades becomes the interval indicated in the specified interval information,
**characterized in that** the slit lamp microscope is configured such that
when the user carries out a predetermined operation using the operating part (104) after adjusting the interval between the slit blades, the controller (101) generates interval information indicating the interval between the slit blades at the time and stores it in the storage (102).

2. The slit lamp microscope according to Claim 1, wherein
the operating part (104) comprises one or more buttons,
the controller (101) associates one piece of the interval information stored in the storage (102) with respect to each of the buttons,
the operation for specifying the interval information is a first operation using the button, and
the predetermined operation is a second operation using the button, which differs from the first operation.

3. The slit lamp microscope according to Claim 1 or 2, wherein
the interval information is associated with patient identification information and stored in the storage (102).

## Patentansprüche

1. Spaltlampenmikroskop (1), mit:
einer Lichtquelle (51), die konfiguriert ist, Beleuchtungslicht zu emittieren;
ein Paar Spaltlamellen, das konfiguriert ist, aus dem Beleuchtungslicht Spaltlicht zu erzeugen;
einem Stellantrieb (200), der konfiguriert ist, eine Antriebskraft zu erzeugen;
einem Mechanismus (210), der konfiguriert ist, den Abstand zwischen den Spaltlamellen beruhend auf der Antriebskraft zu ändern;
einem Speicher (102), der konfiguriert ist, eine oder mehrere Abstandsinformationen zu speichern, die jeweils einen Abstand zwischen den Spaltlamellen angeben;
einem Bedienungsteil (104); und
einer Steuereinrichtung (101), die konfiguriert ist, den Stellantrieb (200) so zu steuern, dass der Abstand zwischen den Spaltlamellen den Abstand annimmt, der in den spezifizierten Abstandsinformation angegeben wird, wenn ein Benutzer eine aus der einen oder den mehreren Abstandsinformationen mittels des Bedienungsteils (104) spezifiziert,
**dadurch gekennzeichnet, dass** das Spaltlampenmikroskop so konfiguriert ist, dass die Steuereinrichtung (101) Abstandsinformationen erzeugt, die den Abstand zwischen den Spaltlamellen zu der Zeit angeben, und sie im Speicher (102) speichert, wenn der Benutzer eine vorgegebene Bedienung mittels des Bedienungsteils (104) nach einer Einstellung des Abstands zwischen den Spaltlamellen ausführt.

2. Spaltlampenmikroskop nach Anspruch 1, wobei
der Bedienungsteil (104) einen oder mehrere Knöpfe aufweist,
die Steuereinrichtung (101) eine im Speicher (102) gespeicherte Abstandsinformation in Bezug auf jeden der Knöpfe verknüpft,
die Bedienung zum Spezifizieren der Abstandsinformationen eine erste Bedienung mittels des Knopfes ist, und
die vorgegebene Bedienung eine zweite Bedienung mittels des Knopfes ist, die sich von der ersten Bedienung unterscheidet.

3. Spaltlampenmikroskop nach Anspruch 1 oder 2, wobei
die Abstandsinformationen mit Patientenidentifikationsinformationen verknüpft und im Speicher (102) gespeichert sind.

## Revendications

1. Microscope à lampe à fente (1), comprenant :
une source lumineuse (51) prévue pour émettre une lumière d'éclairage ;
une paire de lames fendues prévues pour générer une lumière en forme de fente à partir de la lumière d'éclairage ;
un actionneur (200) prévu pour générer une force d'entraînement ;
un mécanisme (210) prévu pour changer l'intervalle entre les lames fendues sur la base de la force d'entraînement ;
une mémoire (102) prévue pour stocker un ou plusieurs éléments d'information d'intervalle indiquant chacun un intervalle entre les lames fendues ;
une groupe d'actionnement (104) ; et
un dispositif de commande (101) prévu pour commander l'actionneur (200) de telle manière que l'intervalle entre les lames fendues devient l'intervalle indiqué dans l'information d'intervalle spécifiée, quand un utilisateur spécifie l'élément d'information d'intervalle, ou un élément parmi les plusieurs éléments d'information d'intervalle en recourant au groupe d'actionnement (104),
**caractérisé en ce que** ledit microscope à lampe à fente est prévu pour que le dispositif de commande (101) génère l'information d'intervalle indiquant l'intervalle entre les lames fendues à ce moment et sauvegarde celle-ci dans la mémoire (102), lorsque l'utilisateur exécute une opération prédéfinie en recourant au groupe d'actionnement (104) après réglage de l'intervalle entre les lames fendues.

2. Microscope à lampe à fente selon la revendication 1, où
le groupe d'actionnement (104) comprend un ou plusieurs boutons,
le dispositif de commande (101) associe un élément de l'information d'intervalle sauvegardé dans la mémoire (102) par rapport à chacun des boutons,
l'opération de spécification de l'information d'intervalle est une première opération recourant au bouton, et
l'opération prédéfinie est une deuxième opération recourant au bouton qui diffère de la première opération.

3. Microscope à lampe à fente selon la revendication 1 ou la revendication 2, où l'information d'intervalle est associée à une information d'identification de patient et sauvegardée dans la mémoire (102).
